# EUROPEAN PATENT APPLICATION

(11) **EP 3 875 060 A1**
(43) Date of publication of application: **08.09.2021**
(21) Application number: 20160335.4
(22) Date of filing: 02.03.2020
(51) Int. Cl.: A61F 2/24

(54) **HEART VALVE PROSTHESIS**

(71) Applicant: Biotronik AG, 8180 Bülach (CH)
(72) Inventor: LUX, Boris, 21037 Hamburg (DE)
(74) Representative: Biotronik Corporate Services SE

(57) **Abstract**

A heart valve prosthesis (1) comprising a valve member (2) having a flexible leaflet arrangement (21) and a valvular support structure (20) for supporting the flexible leaflet arrangement (21), the flexible leaflet arrangement (21) forming at least one flexible leaflet (22A, 22B, 22C) which is movable between an opened position for allowing a blood flow in an antegrade flow direction (F) and a closed position for blocking a blood flow in a retrograde flow direction (R). An anchoring member (3) serves for anchoring the heart valve prosthesis (1) within a patient, the anchoring member (3) comprising a flexible skirt arrangement (31) and an anchoring support structure (30) for supporting the flexible skirt arrangement (31). Herein, the valvular support structure (21) and the anchoring support structure (31) are structurally separate from one another, but are flexibly connected to each other via the flexible leaflet arrangement (21) and the flexible skirt arrangement (31).

## Description

The instant invention relates to a heart valve prosthesis according to the preamble of claim 1.

A heart valve prosthesis of this kind comprises a valve member having a flexible leaflet arrangement and a valvular support structure for supporting the flexible leaflet arrangement, the flexible leaflet arrangement forming at least one flexible leaflet which is movable between an opened position for allowing a blood flow in an antegrade flow direction and a closed position for blocking a blood flow in a retrograde flow direction. An anchoring member serves for anchoring the heart valve prosthesis within a patient, the anchoring member comprising a flexible skirt arrangement and an anchoring support structure for supporting the flexible skirt arrangement.

In typical heart valve prosthesis arrangements, as known for example from US 9,750,603, a flexible leaflet arrangement, for example formed from a pericardial material, is fixed to a support structure in the shape of a stent. The stent forms a lattice structure having a tubular shape and comprises a plurality of closed cells arranged to define a band exhibiting a variable radial stiffness, the flexible leaflet arrangement being placed within a lumen of the stent. The stent herein is formed to self-expand, such that the stent may be delivered to a location of interest in a compressed state, and may expand upon deployment at the location of interest, in particular at the location of the aortic valve within a patient's heart.

There is a desire for a heart valve prosthesis which provides for a high flexibility in adapting to specific conditions at a location of interest, in particular in the vicinity of the aorta for serving as a prosthetic aortic valve. There in addition is a desire for allowing for an easy and flexible delivery and an easy and reliable manufacturing of a heart valve prosthesis.

From DE 10 2017 202 159 A1 a heart valve prosthesis is known which allows for an implantation using a delivery catheter and which comprises a flexible, foldable support structure to which valve leaflets are fixed. The support structure comprises a fixing ring which shall be force-decoupled from the support structure such that a deformation of the fixing ring is possible without a deformation of the support structure upon implantation within a blood vessel of a patient.

It is an object of the instant invention to provide a heart valve prosthesis which allows for a high flexibility in adapting to specific conditions at a location of interest and which further allows for an easy and flexible delivery as well as an easy and reliable manufacturing.

This object is achieved by means of a heart valve prosthesis comprising the features of claim 1.

Accordingly, within the heart valve prosthesis the valvular support structure and the anchoring support structure are structurally separate from one another, but are flexibly connected to each other via the flexible leaflet arrangement and the flexible skirt arrangement.

Within the heart valve prosthesis, a valve member having a valvular support structure and an anchoring member having an anchoring support structure are provided. The valvular support structure and the anchoring support structure herein are structurally separate from one another, such that the valvular support structure and the anchoring support structure are formed by different structural units and as such are not structurally interconnected with each other.

Within the context of the instant text the formation of the valvular support structure and the anchoring support structure as structurally separate units shall be understood to mean that no rigid structural features, such as struts or the like, are formed in between the valvular support structure and the anchoring support structure. The valvular support structure and the anchoring support structure hence are not interconnected with each other by structural elements such as struts, beams or the like extending in between the valvular support structure and the anchoring support structure.

However, a flexible interconnection between the valvular support structure and the anchoring support structure is formed via the flexible leaflet arrangement and the flexible skirt arrangement. In particular, the flexible leaflet arrangement of the valve member and the flexible skirt arrangement of the anchoring member are connected to each other, the flexible leaflet arrangement and the flexible skirt arrangement being formed from a flexible, soft, deformable, pliable material such that by means of the flexible leaflet arrangement and the flexible skirt arrangement no structural interconnection, but a flexible, pliable interconnection between the valvular support structure and the anchoring support structure is formed.

The flexible leaflet arrangement of the valve member and the flexible skirt arrangement of the anchoring member together may be formed integrally in one piece from a pliable material. In another embodiment, the flexible leaflet arrangement of the valve member and the flexible skirt arrangement of the anchoring member may be formed as separate members and may for example be sewed together for providing an interconnection in between the valve member and the anchoring member.

Because the valvular support structure of the valve member and the anchoring support structure of the anchoring member are formed by separate frame-like structures, the heart valve prosthesis upon implantation within a patient may flexibly adapt to specific conditions at a location of interest, for example in the vicinity of the aortic valve and within the aorta. In particular, the positions of the valvular support structure and the anchoring support structure may flexibly be adapted relative to each other, without the anchoring support structure constraining the positioning of the valvular support structure and vice versa. In this way, the anchoring support structure may in particular provide for a fixation to the annulus of an aortic valve, wherein the valvular support structure may be placed within the aorta and may be adapted to the specific shape of the aorta.

The heart valve prosthesis of the invention hence, upon implantation, may flexibly adapt to conditions at an implantation site within a patient.

In addition, because the valve member and the anchoring member are formed with separate support structures, delivery may be eased in that, e.g., a delivery catheter may have a reduced bending stiffness and hence may be passed through the aortic bend in an easy manner.

During delivery, the placement of the anchoring member and the valve member may take place in a multi-step process, in which e.g. first the anchoring member is deployed and placed for example at the annulus of the aortic valve and e.g. in a second step the valve member is deployed by placing the valve member within the aorta of the patient.

In one aspect, the flexible leaflet arrangement and/or the flexible skirt arrangement are made from a biocompatible material, in particular from a biocompatible pericardial material of animal origin, optionally being a dry material, preferably a bovine, porcine, horse or kangaroo pericardial material, from a material based on bacterial cellulose, or from a polymeric material. Most preferred may be, in one embodiment, a porcine pericardial material, e.g. a dry porcine pericardial material.

The flexible leaflet arrangement and the flexible skirt arrangement, herein, may be made from the same material, for example from a pericardial (dry) material or a polymeric material. Alternatively, the flexible leaflet arrangement and the flexible skirt arrangement may be made from different materials, one for example being formed from a pericardial (dry) material and the other from a polymeric material. The latter allows for an adaption of the leaflet arrangement and the skirt arrangement to specific needs and conditions, in particular to a specific arrangement, for example the leaflet arrangement, to requirements for a flexibility, uniformity and elasticity, the skirt arrangement potentially having a reduced requirements in this respect.

In one aspect, the flexible skirt arrangement forms an inflow edge, wherein at least a portion of the flexible skirt arrangement is conically tapered in the antegrade flow direction beyond the inflow edge. The anchoring member hence, due to the shaping of the skirt arrangement, assumes a conical shape, the anchoring member tapering in the antegrade flow direction towards the valve member.

In another aspect, the flexible leaflet arrangement may form an outflow edge, wherein at least a portion of the flexible leaflet arrangement is conically tapered in the retrograde flow direction beyond the outflow edge. Hence, the valve member, due to the shaping of the leaflet arrangement, assumes a conical shape, the valve member being tapered in the retrograde flow direction and hence towards the anchoring member.

The conical shape of the valve member may provide for a beneficial force introduction and support in particular in a closed position of the leaflet arrangement for blocking a flow in the retrograde flow direction. In case of a retrograde flow, the leaflets of the leaflet arrangement are moved from an opened position into a closed position, in which the leaflets are flexibly bent inwards and abut each other in order to close a flow path. Due to the conical shape of the valve member, herein, a deformation of the valve member, in particular the valvular support structure (which may be pulled inwards due to forces acting onto the leaflets of the leaflet arrangement) is reduced, the conical shape providing for a structurally beneficial force introduction and a reduced load acting onto the valvular support structure.

In addition, the valve member may be to some extent deformed by the deployment at the implantation site, for example within the aorta, such that the valvular support structure is partially compressed due to the placement at the implantation site. Such deformation may cause a drapery in the leaflet arrangement. In case of a blocking of flow in the retrograde flow direction in the closed position of the leaflets of the leaflet arrangement, a loading on the valve member first causes a reduction in the drapery in the leaflet arrangement, before a substantial load acts onto the valvular support structure.

In one aspect, both the anchoring member and the valve member have a conical shape, the skirt arrangement of the anchoring member and the leaflet arrangement of the valve member being connected to each other at a waist of the heart valve prosthesis, the waist corresponding to a location, when viewed along the antegrade flow direction, at which the heart valve prosthesis comprises a minimum diameter (measured transverse to the antegrade flow direction).

In one aspect, the valvular support structure is formed by a ring element. The ring element may be circumferentially closed, or may be opened at a circumferential location.

The ring element may be formed from a wire. The wire may be circumferentially closed, the ring element hence being formed by a closed loop. Alternatively, the wire forming the ring element may have two ends, wherein the ring element may be opened at one circumferential position, or the ends of the wire may be fixedly connected to each other such that a closed loop is formed.

In one embodiment, the ring element is formed by a continuous element having no branch-offs. The valvular support structure hence does not form any cells and does not form any nodes from which multiple (more than two) strut sections extend.

In one embodiment, the valvular support structure may have a meandering or crown-like shape, the valvular support structure being bent such that upper vertices alternate with lower vertices, the upper vertices being arranged in the vicinity of an outflow end of the valve member, wherein the lower vertices point towards the anchoring member.

The valvular support structure, in one aspect, may have a conical shape, the lower vertices being displaced radially inwards with respect to the upper vertices.

In one aspect, the valvular support structure forms at least one curved section for supporting the flexible leaflet arrangement, the at least one curved section being associated with the at least one flexible leaflet for defining a bending line about which the at least one flexible leaflet is flexibly bendable for a movement between the opened position and the closed position. The number of curved sections of the valvular support structure, in one embodiment, matches the number of leaflets of the leaflet arrangement. In particular, each leaflet of the leaflet arrangement may be associated with a curved section of the valvular support structure, such that each curved section defines a bending line for the associated leaflet of the flexible leaflet arrangement.

Herein, the curved sections may for example have a parabolic shape, each curved section extending in between a pair of upper vertices of the valvular support structure and comprising a lower vertex arranged in between the pair of upper vertices. Each curved section hence, with its lower vertex, points towards the anchoring member.

In one aspect, the valvular support structure forms at least one tip section, wherein the flexible leaflet arrangement is fixed to the at least one tip section. The number of tip sections formed by the valvular support structure may for example match the number of leaflets of the flexible leaflet arrangement. A tip section herein may for example be formed in between two adjacent leaflets of the leaflet arrangement. For example, the tip section may be formed at the location of an upper vertex of the valvular support structure, the valvular support structure for example having a meandering or crown-like shape.

The valvular support structure may comprise one tip section, but may, in another embodiment, also comprise more than one tip section. For example, the valvular support structure may comprise 2, 3, 4 or 5 tip sections, wherein it also is conceivable that the valvular support structure comprises more than 5 tip sections.

The number of tip sections may, in one embodiment, correspond to the number of leaflets of the flexible leaflet structure.

The fixing of the flexible leaflet arrangement to the at least one tip section of the valvular support structure may take place in different ways.

For example, the flexible leaflet arrangement may be tightly hang-up on the at least one tip section. In this case, for example, a bag-like structure is formed on the flexible leaflet arrangement, the tip section engaging with the bag-like structure such that the flexible leaflet arrangement is hung up on the tip sections of the valvular support structure.

In another embodiment, the flexible leaflet arrangement forms at least one fastening section adjacent, when viewed along a circumferential direction about the antegrade flow direction, to the at least one flexible leaflet of the leaflet arrangement, wherein the flexible leaflet arrangement is fixed to the tip section via the at least one fastening section.

The at least one fastening section may, for example, comprise at least one flap section which, for fixing the flexible leaflet arrangement to the tip section, is folded about a beam section of the valvular support structure at the tip section, wherein for example two flap sections of the at least one fastening section may be sewed together in the vicinity of the tip section for fixing the flexible leaflet arrangement to the tip section.

In one aspect, the anchoring support structure is formed by a ring element. The ring element may be circumferentially closed, or may be opened at a circumferential location.

The ring element may be formed from a wire. The wire may be circumferentially closed, the ring element hence being formed by a closed loop. Alternatively, the wire forming the ring element may have two ends, wherein the ring element may be opened at one circumferential position, or the ends of the wire may be fixedly connected to each other such that a closed loop is formed.

In one embodiment, the ring element forming the anchoring support structure is formed by a continuous element having no branch-offs. The anchoring support structure hence does not form any cells and does not form any nodes from which multiple (more than two) strut sections extend.

In one aspect, the anchoring support structure may have a meandering shape, upper vertices and lower vertices of the anchoring support structure hence alternating with each other, the upper vertices being displaced with respect to the lower vertices along the antegrade flow direction.

In one aspect, the anchoring support structure comprises a multiplicity of radially outer portions and a multiplicity of radially inner portions, wherein, when viewed along a circumferential direction about the antegrade flow direction, the radially outer portions alternate with the radially inner portions. The radially outer portions are displaced with respect to the radially inner portions radially towards the outside.

The radially outer portions, in particular, may serve to provide for an abutment to native structure of a patient, for example in the region of the annulus of the aortic valve of the patient. Hence, by means of the radially outer portions a fixation of the anchoring member to native structure or, possibly, to a prior prosthetic valve which shall be replaced by the heart valve prosthesis is provided.

The radially inner portions, in particular, may serve for fixing the skirt arrangement of the anchoring member to the anchoring support structure, the skirt arrangement for example being sewed to the radially inner portions of the anchoring support structure.

The flexible skirt arrangement, in one embodiment, may for example be fixed to the anchoring support structure by means of a seam circumferentially extending about the anchoring support structure.

In one embodiment, the multiplicity of radially outer portions is displaced, along the antegrade flow direction, with respect to said multiplicity of radially inner portions. The radially outer portions may for example comprise upper vertices of the anchoring support structure, the radially inner portions in turn comprising lower vertices of the anchoring support structure.

In one aspect, the flexible skirt arrangement comprises a body and at least one folding section formed on the body. The at least one folding section is folded, at a fold line, with respect to the body to reach around the anchoring support structure. The at least one folding section may for example have a flap-like shape, the at least one folding section being folded with respect to the body of the skirt arrangement such that the at least one folding section reaches around the anchoring support structure, for example in the vicinity of a lower vertex of the anchoring support structure, for fixing the flexible skirt arrangement to the anchoring support structure.

In one embodiment, the flexible skirt arrangement comprises multiple folding sections, each of the folding sections being associated with a lower vertex of the anchoring support structure and being folded around an associated lower vertex of the anchoring support structure for fixing the skirt arrangement circumferentially to the anchoring support structure.

In one embodiment, the at least one folding section may be folded with respect to the body such that the at least one folding section is placed radially outside of the body.

In one aspect, the flexible leaflet arrangement comprises a first joining edge and the flexible skirt arrangement comprises a second joining edge, wherein the first joining edge and the second joining edge are connected to each other by a seam. The flexible leaflet arrangement and the flexible skirt arrangement are formed, in one embodiment, from separate units of flexible, pliable material, the flexible leaflet arrangement and the flexible skirt arrangement being interconnected which each other by sewing the leaflet arrangement and the skirt arrangement together.

In one embodiment, the first joining edge and/or the second joining edge comprise a meandering shape, joining flaps alternating with recesses along a circumferential direction about the antegrade flow direction. In case both the first joining edge and the second joining edge comprise a meandering shape, joining flaps of the first joining edge and joining flaps of the second joining edge may be arranged with respect to each other such that the joining flaps of the first joining edge and the joining flaps of the second joining edge interleave with each other. The joining flaps, in one embodiment, may point towards the outside, such that the joining flaps are arranged radially outside of a body of the skirt arrangement and a body of the flexible leaflet arrangement.

In one aspect, the seam for interconnecting the flexible skirt arrangement and the flexible leaflet arrangement to each other is formed by at least one thread forming first thread sections extending between neighboring first joining flaps, and forming second thread sections extending between neighboring second joining flaps.

In one embodiment, said first thread sections and said second thread sections are arranged substantially parallel to one another.

In one embodiment, said first thread sections and said second thread sections are displaced with respect to each other along the antegrade flow direction.

In one embodiment, said first thread sections and said second thread sections are arranged at a side of the heart valve prosthesis facing radially outwards.

In one embodiment, the at least one thread forms third thread sections and fourth thread sections arranged at an angle with respect to one another. Said third thread sections and said fourth thread sections, in one embodiment, are arranged at a side of the heart valve prosthesis facing radially outwards.

In particular, the entire thread comprised of first, second, third and fourth thread sections may be placed outside of an inner lumen of the heart valve prosthesis, such that the thread does not interfere with a blood flow though the inner lumen.

In one embodiment, at least some of the first thread sections are adjoined by a third thread section and a fourth thread section.

In one embodiment, at least some of the second thread sections are adjoined by a third thread section and a fourth thread section.

The thread sections may together form a seam having a substantially trapezoidal shape, the thread sections forming trapezoids being for example opened and one or two edges. Because in one embodiment the thread sections are arranged at an outside of the heart valve prosthesis, at which also for example the joining flaps of the flexible leaflet arrangement and the flexible skirt arrangement come to rest, the seam may be formed such that the joining flaps flatly about a body of the flexible leaflet arrangement respectively a body of the flexible skirt arrangement.

In one aspect, the connection of the valvular support structure and the anchoring support structure via the flexible leaflet arrangement and the flexible skirt arrangement is releasable in an implanted state of the heart valve prosthesis.

By providing a releasable connection in between the valvular support structure and the anchoring support structure, in one embodiment, it may be achieved that the valve member and the anchoring member may be disconnected from each other for example for replacing the valve member in an implanted state of the heart valve prosthesis. In particular, in case of a failure or in case of another replacement need the valve member may be disconnected from the anchoring member and hence may be replaced by a replacement valve member while the anchoring member remains in place within the patient.

In another embodiment, the releasable connection of the valve member and the anchoring member also makes it possible that for implantation for example first the anchoring member and, in another implantation step, the valve member is implanted, wherein the anchoring member and the valve member are connected to each other only within the patient, and hence while for example the anchoring member already is implanted.

The connection in between the valve member and the anchoring member, that is in between the valvular support structure of the valve member and the anchoring support structure of the anchoring member, is achieved via the flexible leaflet arrangement and the flexible skirt arrangement. For achieving a releasable connection, herein, for example joining flaps at the meander-shaped joining edges of the flexible leaflet arrangement and the flexible skirt arrangement may be brought into engagement with each other for connecting the flexible leaflet arrangement and the flexible skirt arrangement to each other, wherein the connection may be releasable by releasing the engagement in between the joining flaps, for example by exerting a predefined movement in between the valve member and the anchoring member.

The joining flaps for example may provide for a connection in the manner of a bayonet lock.

The joining flaps may be formed in a rigid manner or may comprise rigid sections such that a reliable, positive locking connection may be achieved via the joining flaps. To provide for rigid sections, for example rigid elements may be sewed to the joining flaps to at least partially reinforce the joining flaps.

Alternatively or in addition, the joining flaps may be formed from a different material than the flexible leaflet arrangement and/or the flexible skirt arrangement, or may comprise a different material thickness, in particular an increased material thickness in comparison to other portions of the flexible leaflet arrangement and/or the flexible skirt arrangement. The material of the joining flaps in particular may provide for an increased rigidity in comparison to other portions of the flexible leaflet arrangement and/or the flexible skirt arrangement.

A releasable connection may also be provided by other means, for example by elements connected to the joining edges of the flexible leaflet arrangement and the flexible skirt arrangement, in particular locking elements providing for a positive locking connection. Such locking elements may for example have a ring shape and may provide for a locking connection in the manner of a bayonet lock.

The object of the invention shall subsequently be explained in more detail with reference to the embodiments shown in the figures. Herein:
- Fig. 1: shows a drawing of an embodiment of a heart valve prosthesis comprising a valve member and an anchoring member;
- Fig. 2: shows a drawing of the valve member and the anchoring member in a separate state;
- Fig. 3: shows a separate view of the valve member;
- Fig. 4: shows a view of a valvular support structure of the valve member;
- Fig. 5: shows a view of the valve member, in a closed position of leaflets of a flexible leaflet arrangement;
- Fig. 6: shows a separate view of the anchoring member;
- Fig. 7: shows a view of an anchoring support structure of the anchoring member;
- Fig. 8: shows a view of an embodiment of a piece of material forming the flexible leaflet arrangement of the valve member;
- Fig. 9: shows a view of an embodiment of a piece of material forming a single leaflet of the flexible leaflet arrangement;
- Fig. 10: shows a view of a fastening section of the flexible leaflet arrangement;
- Fig. 11: shows an embodiment of a piece of material forming the flexible skirt arrangement;
- Fig. 12: shows a view of an embodiment of the anchoring member in the region of an inflow edge illustrating the fixation of the skirt arrangement to the anchoring support structure;
- Fig. 13: shows another view of the embodiment of the anchoring member according to Fig. 12;
- Fig. 14: shows a schematic view of the anchoring member of the embodiment of Fig. 12;
- Fig. 15: shows a schematic view of the anchoring member, illustrating a force distribution in an implanted state of the anchoring member;
- Fig. 16A: shows a view of a connection of wire ends of a wire forming an anchoring support structure or a valvular support structure;
- Fig. 16B: shows a view of another embodiment of a connection of wire ends of a wire forming an anchoring support structure or a valvular support structure;
- Fig. 17: shows a view of an embodiment of a valve member, illustrating seams for closing the flexible leaflet arrangement and the flexible skirt arrangement as well as for connecting the flexible leaflet arrangement and the flexible skirt arrangement to each other;
- Fig. 18: shows a view of an embodiment of a piece of material for forming the flexible leaflet arrangement;
- Fig. 19: shows a view of joining edges of the flexible leaflet arrangement and the flexible skirt arrangement for interconnecting the flexible leaflet arrangement and the flexible skirt arrangement;
- Fig. 20: shows a view of the joining edges in an interconnected state, from an inside of the valve (which is the opposite from Fig. 22 below; showing from an outside of the valve; i.e. between the valve and aorta)
- Fig. 21: shows a view of a seam for interconnecting the flexible skirt arrangement and the flexible leaflet arrangement;
- Fig. 22: shows another view of the seam, from an outside of the valve; i.a. a flat cut view from the top, which shows that the parts 26 and 36 are seamed together; The complete seam is between the valve and the aorta and therefore hidden for the blood flow ;
- Fig. 23: shows yet another view of the seam;
- Fig. 24: illustrates a step for manufacturing a valve member;
- Fig. 25: shows a view of the flexible leaflet arrangement of the valve member during manufacturing;
- Fig. 26: illustrates the placement of a valvular support structure within the flexible leaflet arrangement;
- Fig. 27: illustrates the fixing of the flexible leaflet arrangement to the valvular support structure;
- Fig. 28: further illustrates the fixing of the flexible leaflet arrangement to the valvular support structure;
- Fig. 29: provides a side view of the arrangement of Fig. 28;
- Fig. 30: illustrates the flexible leaflet arrangement fixed to the valvular support structure;
- Fig. 31: illustrates a sewing of a fastening section to the valvular support structure;
- Fig. 32: shows a schematic view of the flexible leaflet arrangement fixed to the valvular support structure;
- Fig. 33: shows a step in manufacturing an anchoring member;
- Fig. 34: illustrates another step in manufacturing the anchoring member;
- Fig. 35: illustrates the fixing of a flexible skirt arrangement of the anchoring member to an anchoring support structure;
- Fig. 36: illustrates a seam for fixing the anchoring support structure to the flexible skirt arrangement;
- Fig. 37: shows a schematic view of the seam for connecting the flexible skirt arrangement and the anchoring support structure;
- Fig. 38: shows another step in manufacturing the anchoring members;
- Fig. 39: shows the anchoring member prior to connecting it to the valve member;
- Fig. 40: illustrates the interconnection in between the flexible skirt arrangement and the anchoring support structure; and
- Fig. 41: shows the first heart valve prosthesis with a valve member connected to the anchoring member.

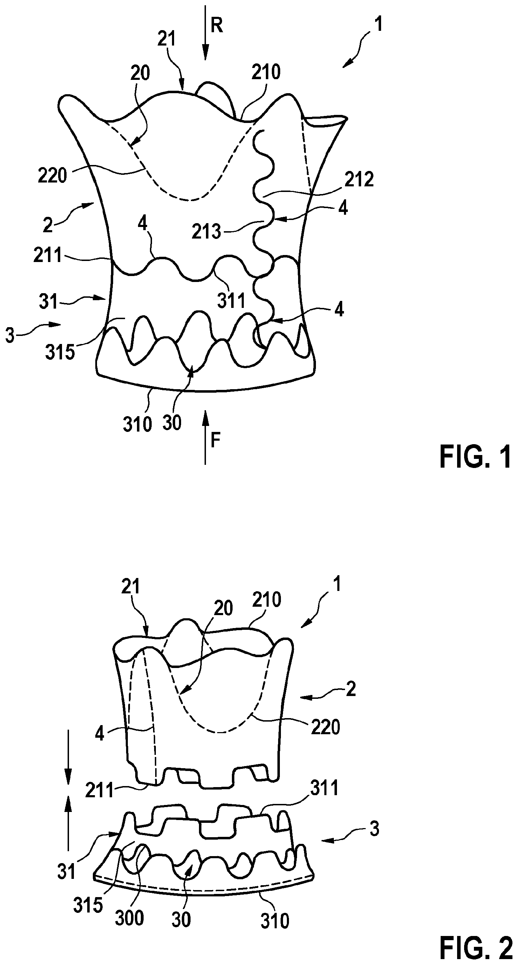

Figs. 1 and 2 show an embodiment of a heart valve prosthesis 1 comprising a valve member 2 and an anchoring member 3.

The valve member 2 comprises a flexible leaflet arrangement 21 supported by a valvular support structure 20. The anchoring member 3 comprises a flexible skirt arrangement 31 supported by an anchoring support structure 30. The valve member 2 and the anchoring member 3 are interconnected with each other at joining edges 211, 311 of the flexible leaflet arrangement 21 and the flexible skirt arrangement 31 by means of a seam 4, such that a valve arrangement is formed allowing for a prosthetic replacement of a native heart 12 of a patient, for example the aortic valve.

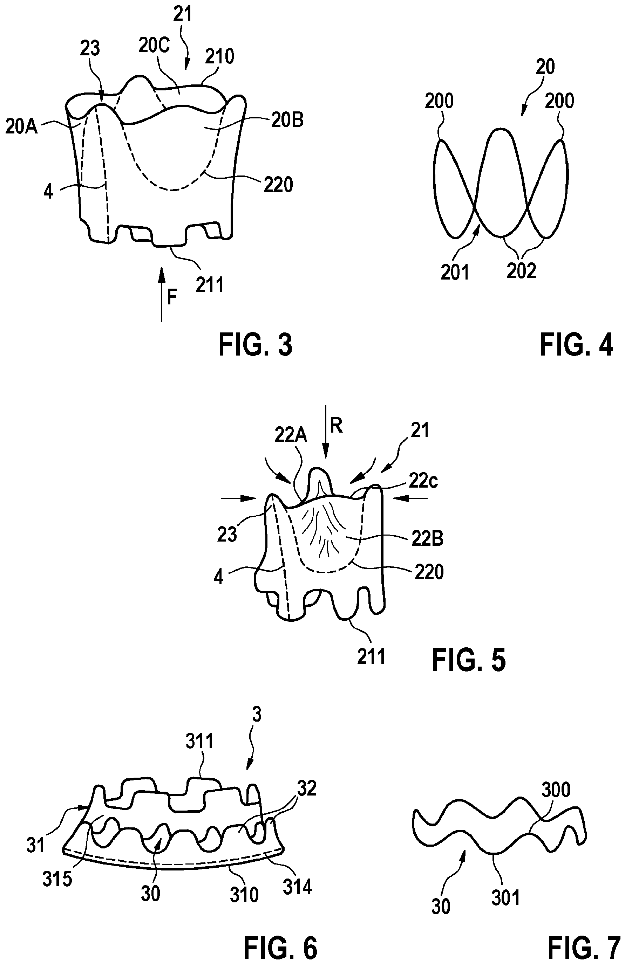

Referring now to Figs. 3 to 5, the flexible leaflet arrangement 21 of the valve member 2 forms a multiplicity of leaflets 22A, 22B, 22C, which are flexibly movable with respect to the valvular support structure 20 for opening or closing the heart valve prosthesis 1. In particular, the leaflets 22A, 22B, 22C may assume an opened position in which the leaflets 22A, 22B, 22C are spaced apart from one another and form an opening therebetween, such that a flow may pass through the valve member 2 in an antegrade flow direction F, as illustrated in Fig. 3. In addition, the leaflets 22A, 22B, 22C may assume a closed position, as illustrated in Fig. 5, in which the leaflets 22A, 22B, 22C are moved to approach and abut one another, such that by means of the leaflets 22A, 22B, 22C a flow path in a reverse, retrograde flow direction R is closed and hence a flow passage is blocked by the heart valve prosthesis 1.

The valvular support structure 20, as illustrated in Fig. 4, has a meandering or crown-like shape and is received within the flexible leaflet arrangement 21 of the valve member 2. The valvular support structure 20 serves to structurally support the flexible leaflet arrangement 21, such that the leaflets are spatially spanned by means of the valvular support structure 20 and may flexibly move in between their opened position (Fig. 3) and their closed position (Fig. 5) in order to allow a flow in the antegrade flow direction F, but to reliably block a flow in the retrograde flow direction R.

As visible from Fig. 4, the valvular support structure 20 comprises lower vertices 202 and upper vertices 200, which alternate along a circumferential direction about the antegrade flow direction F and the retrograde flow direction R. The upper vertices 200 are arranged in the vicinity of an outflow edge 210 formed by the flexible leaflet arrangement 21 and are displaced with respect to the lower vertices 202 along the antegrade flow direction F and the retrograde flow direction R. By means of the lower vertices 202, the valvular support structure 20 points towards the anchoring member 3.

The valvular support structure 20, in the shown embodiment, is formed by a ring element which forms a circumferentially closed loop. The ring element is circumferentially closed and does not comprise any branch-offs in that no nodes are formed by the ring element from which more than two structural strut sections extend. Rather, the ring element continuously extends in a meandering or crown-like shape to form the valvular support structure 20.

The valve member 2 comprises a conical shape, the valve member 2 tapering in the retrograde flow direction R towards the anchoring member 3. The conical shape is defined by the shape of the flexible leaflet arrangement 21, which tapers towards the anchoring member 3. The conical shape in addition is defined by the shape of the valvular support structure 20, which likewise is tapered towards the anchoring member 3 in that the lower vertices 202 are displaced radially inwards with respect to the upper vertices 200, the valvular support structure 20 hence also tapering in the retrograde flow direction R.

Due to the conical shape of the valve member 2, a load acting onto the valve member 2 in the closed position of the leaflets 22A, 22B, 22C, caused by a retrograde flow acting onto the leaflet arrangement 21, may beneficially be taken up by the valvular support structure 20 and beneficially does not cause a substantial deformation of the valve member 2. In particular, due to the conical shape, a load force acting on the upper vertices 200 of the valvular support structure 20 by a pulling action of the flexible leaflet arrangement 21 is counteracted by the tapering shape of the valvular support structure 20.

The valvular support structure 20 forms curved sections 201 which may have a substantially parabolic shape and extend in between a pair of adjacent upper vertices 200. Each curved section 201 herein forms a respective lower vertex 202 arranged circumferentially in between the pair of associated upper vertices 200.

Each curved section 201 is associated with a leaflet 22A, 22B, 22C, the curved sections 201 defining bending lines 220 for the associated leaflets 22A, 22B, 22C, such that each leaflet 22A, 22B, 22C may bend in a defined manner about an associated bending line 220 when moving in between its opened position (Fig. 3) and the closed position (Fig. 5).

The valve member 2, upon implantation at an implantation site in a patient, may flexibly adapt to the specific conditions at the implantation site. In particular, the valvular support structure 20 may elastically deform to adapt to the shape of for example an aorta in which the valve member 2 is placed.

The valve member 2 is joined to the anchoring member 3 at the joining edges 211, 311 of the leaflet arrangement 21 and the skirt arrangement 31 by means of a seam 4. Herein, no structural interconnection in between the valvular support structure 20 and the anchoring support structure 30 exists, the valvular support structure 20 and the anchoring support structure 30 being formed by separate structural entities, with no structural features such as struts or the like extending in between the valvular support structure 20 and the anchoring support structure 30. A connection in between the valve member 2 and the anchoring member 3 is formed solely via the pliable material of the leaflet arrangement 21 and the skirt arrangement 31.

The flexible interconnection in between the valve member 2 and the anchoring member 3 via the pliable material of the leaflet arrangement 21 and the skirt arrangement 31 allows for a flexible, adaptable implantation of the valve member 2 and the anchoring member 3 at an implantation site, wherein the valve member 2 and the anchoring member 3 separately may adapt to specific conditions at the implantation site. In particular, the valvular support structure 20 and the anchoring support structure 30 may elastically deform independent of each other, such that the valvular support structure 20 may for example adapt to a specific shape of native structure within the aorta of a patient, whereas the anchoring member 3 may be placed for example within the annulus of an aortic valve of the patient, the anchoring support structure 30 being able to adapt to the specific shape of the annulus upon implantation.

In addition, due to the structurally separate formation of the valvular support structure 20 and the anchoring support structure 30, a delivery by means of a delivery catheter in a multi-step process becomes possible. In particular, the anchoring member 3 and the valve member 2 may be placed at an implantation site in different delivery steps, allowing for example to first deploy the anchoring member 3 within the annulus of a patient's aortic valve and to then, in another step, deploy the valve member 2 within the aorta of the patient.

Referring now to Figs. 6 and 7, the anchoring member 3 is formed by the skirt arrangement 31 and the anchoring support structure 30 fixedly connected to the skirt arrangement 31.

The anchoring support structure 30 comprises a meandering shape having lower vertices 301 and upper vertices 300 which alternate along a circumferential direction about the antegrade flow direction F and the retrograde flow direction R. The anchoring support structure 30 is formed by a ring element forming a circumferentially closed loop, the anchoring support structure 30 being fixed to the flexible skirt arrangement 31 in the region of the lower vertices 301, as this is visible from Fig. 7.

The flexible skirt arrangement 31 comprises a body 315 and folding sections 32 having a flap-like shape. The folding sections 32 are folded about a fold line 314 with respect to the body 315 at an inflow edge 310 of the anchoring member 3, the folding sections 32 reaching around the anchoring support structure 30 at the lower vertices 301 of the anchoring support structure 30.

The folding sections 32 are folded with respect to the body 315 such that the folding sections 32 are placed radially outwards of the body 315, as visible from Fig. 6.

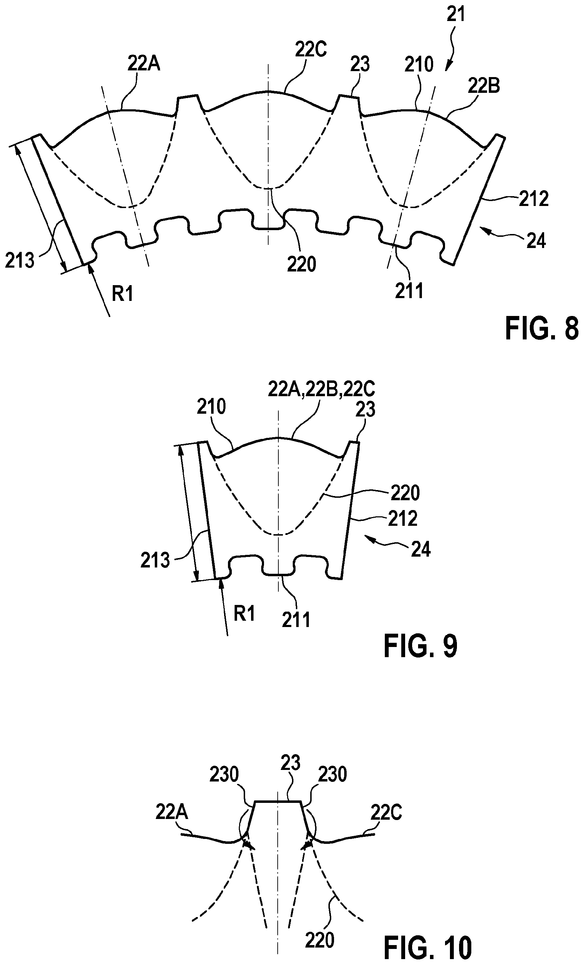

Referring now to Fig. 8, the flexible leaflet arrangement 21 of the valve member 2 may be formed by a single piece of a flexible, pliable material 24. In this embodiment, the leaflets 22A, 22B, 22C are formed from the same piece of material, wherein the flexible leaflet arrangement 21 is formed by sewing lateral edges 212, 213 of the piece of material 24 together to form a circumferentially closed structure, as visible from Figs. 3 and 5.

In an alternative embodiment, the leaflets 22A, 22B, 22C may be formed from separate pieces of material 24, as illustrated in Fig. 9. Herein, the separate pieces 24 associated with the leaflets 22A, 22B, 22C are sewed together at edges 212, 213 to form a circumferentially closed structure to form a leaflet arrangement 21 similar to the one shown in Figs. 3 and 5.

In each case, the material may be a biocompatible material, for example a biocompatible pericardial material of animal origin (e.g. dry pericardial material) such as a bovine, porcine, horse or kangaroo pericardial material (e.g. dry), or a material based on bacterial cellulose or a biocompatible polymeric material. In certain instances, the leaflets 22A, 22B, 22C may be formed from pre-shaped leaflets made from one or more of the aforementioned materials; e.g. 3D-printed leaflets made from a biocompatible polymeric material.

Thus, in one embodiment, independent on whether the leaflets 22A, 22B, 22C are formed from separate pieces or are formed from a single piece of material 24, the leaflets 22A, 22B, 22C may be pre-shaped such that they e.g. do not have a flap shape, but may have a pre-shaped, curved shape. In this way the leaflets 22A, 22B, 22C may for example form bags for trapping a blood flow in a retrograde flow direction. By pre-shaping the leaflets 22A, 22B, 22C to cause the leaflets 22A, 22B, 22C to assume e.g. a curved shape, a retrograde flow may cause the leaflets 22A, 22B, 22C to be pressed against each other such that a tightness in between the leaflets 22A, 22B, 22C may be improved in a closed position of the leaflets 22A, 22B, 22C. At the same time, a loading on the valvular support structure 20 may be reduced in that pressure forces acting on the leaflets 22A, 22B, 22C caused by a retrograde blood flow are substantially not or at least less redirected into radial forces acting on the valvular support structure 20, which otherwise may potentially cause pulling forces on the valvular support structure 20 causing the valvular support structure 20 to be pulled radially inwards.

A pre-shaping of the leaflets 22A, 22B, 22C may further cause a soft abutment of abutment edges of the leaflets 22A, 22B, 22C. In particular when the leaflets 22A, 22B, 22C move inwards to block a retrograde flow when the heart valve prosthesis is transferred from the open state to the closed state, the leaflets 22A, 22B, 22C may becaused to softly come into abutment with each other, hence avoiding a hard flapping of the leaflets 22A, 22B, 22C.

The flexible leaflet arrangement 21 forms, in between adjacent leaflets 22A, 22B, 22C, fastening sections 23 which serve to fixedly connect the flexible leaflet arrangement 21 to the valvular support structure 20. The fastening sections 23 may for example, as illustrated in Fig. 10, form flap sections 230, which may be folded to form for example a bag-like structure which may engage with end sections at the upper vertices 200 of the valvular support structure 20, or may be sewed to beam sections extending from the upper vertices 200 of the valvular support structure 20.

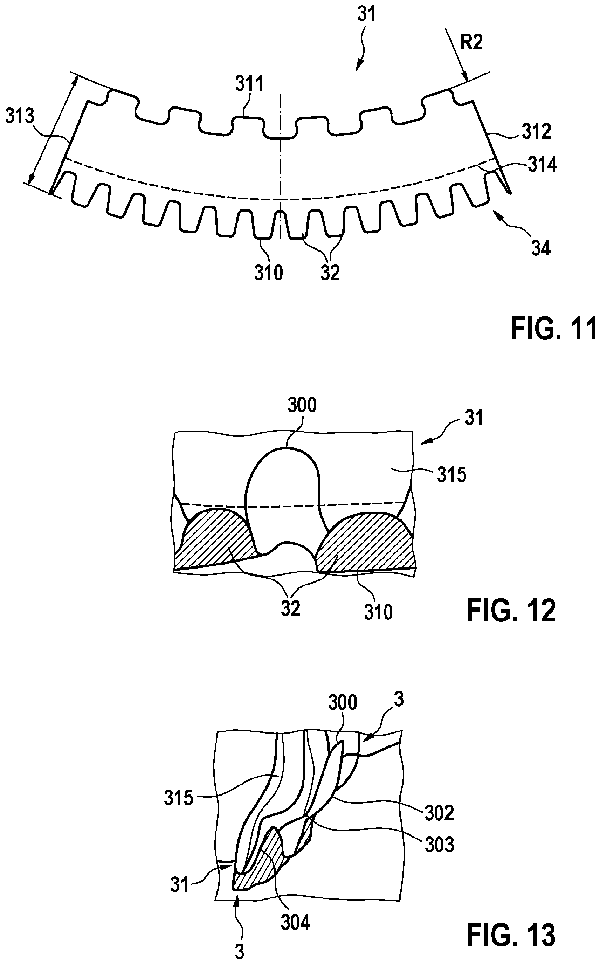

Referring now to Fig. 11, the skirt arrangement 31 of the anchoring member 3 may be formed from a single piece of flexible, pliable material 34, the piece of material 34 being sewed together at lateral edges 312, 313 to form a circumferentially closed structure.

In an alternative embodiment, the skirt arrangement 31 may be formed from multiple pieces of material 34, the pieces of material 34 being sewed together to again form a circumferentially closed structure.

Referring now to Figs. 12 to 14, in one embodiment the anchoring support structure 30 may have a meandering shape being formed by radially outer portions 302 and radially inner portions 304 which are adjoined by means of linking portions 303. The radially outer portions 302 comprise the upper vertices 300 and are displaced radially with respect to the radially inner portions 304 towards the outside, the radially inner portions 304 comprising the lower vertices 301.

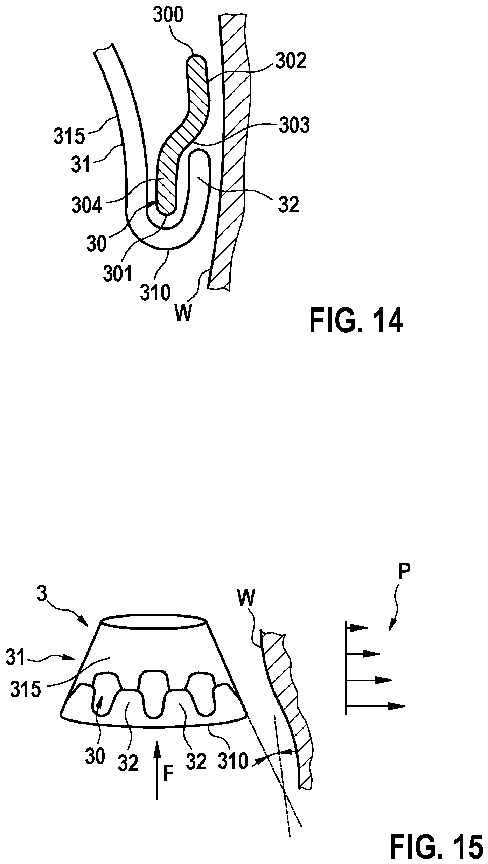

In this embodiment, the radially outer portions 302, which circumferentially alternate with the radially inner portions 304, may serve to provide for a seating of the anchoring member 3 on native structure at an implantation site, for example on a wall section W in the region of an annulus of an aortic valve of a patient, as illustrated in Fig. 14.

The radially inner portions 304, in turn, serve for supporting the flexible skirt arrangement 31, the flexible skirt arrangement 31 being fixed to the radially inner portions 304 by means of the folding sections 32, which are folded around the radially inner portions 304, as visible from Fig. 12.

As visible from Fig. 1 in combination with Fig. 15, the anchoring member 3 may have a generally conical shape, the anchoring member 3 tapering in the antegrade flow direction F and hence towards the valve member 2. As illustrated in Fig. 15, due to the conical shape a pressure P acting in between the anchoring member 3 and native structure, in particular a wall section W at the implantation site, may vary along the antegrade flow direction X, the pressure P in between the anchoring member 3 and the wall section W being largest in the region of the inflow edge 310, wherein the distribution of the pressure P depends on the conical angle of the anchoring member 3.

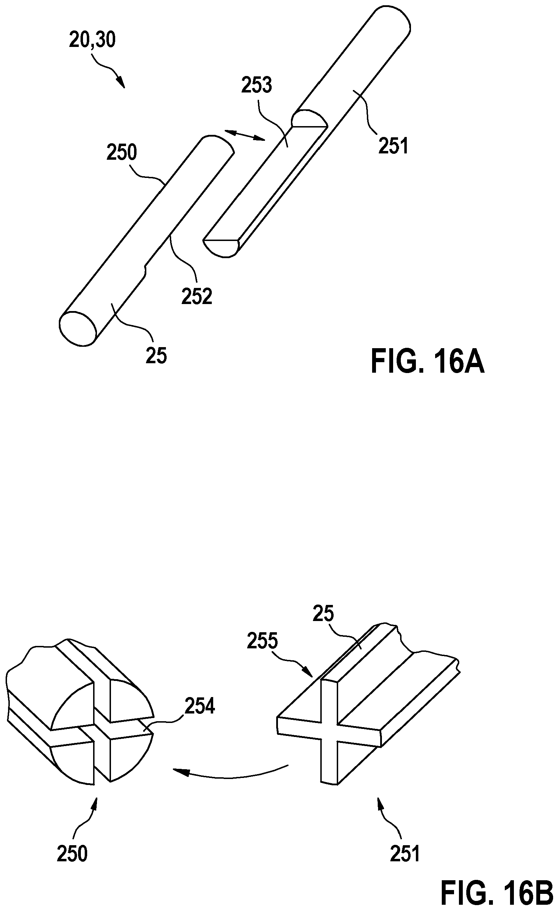

Referring now to Fig. 16A, the valvular support structure 20 as well as the anchoring support structure 30 may be formed by a wire 25 which may be bent to assume a meandering or crown-like shape, as visible from Fig. 4, respectively Fig. 7. The valvular support structure 20 as well as the anchoring support structure 30 herein are formed in each case by a circumferentially closed ring element, which for example may be formed by a wire 25 which continuously extends circumferentially to form a closed loop structure.

Herein, the wire 25 forming the valvular support structure 20 or the anchoring support structure 30 may be integrally closed in the circumferential direction. Alternatively, the wire 25 may have ends 250, 251 which, for forming the valvular support structure 20 respectively the anchoring support structure 30, may be connected to each other for example by an adhesive bond, for example by gluing, soldering or welding, or by a positive-fit or press-fit connection, for example by means of an additional fitting sleeve or the like fitted on the wire 25. At the ends 250, 251 of the wire 25, flattened sections 252, 253 may be formed, such that the ends 250, 251 may be placed on one another and may be joined to each other without increasing the cross-sectional diameter of the wire 25.

An embodiment of a positive-fit connection of wire ends 250, 251 of a wire 25 is illustrated in Fig. 16B. For example, one wire end 250 of the wire 25 may comprise a slot arrangement 254, comprised for example of two perpendicular slots, into which the other wire end 251 may be inserted by means of a positive locking arrangement 255 having a cross shape. By engaging the wire ends 250, 251 with each other, a positive locking connection in between the wire ends 250, 251 may be obtained, wherein the engagement in between the wire ends 250, 251 may in addition form a press-fit connection such that the wire ends 250, 251 are securely held on each other.

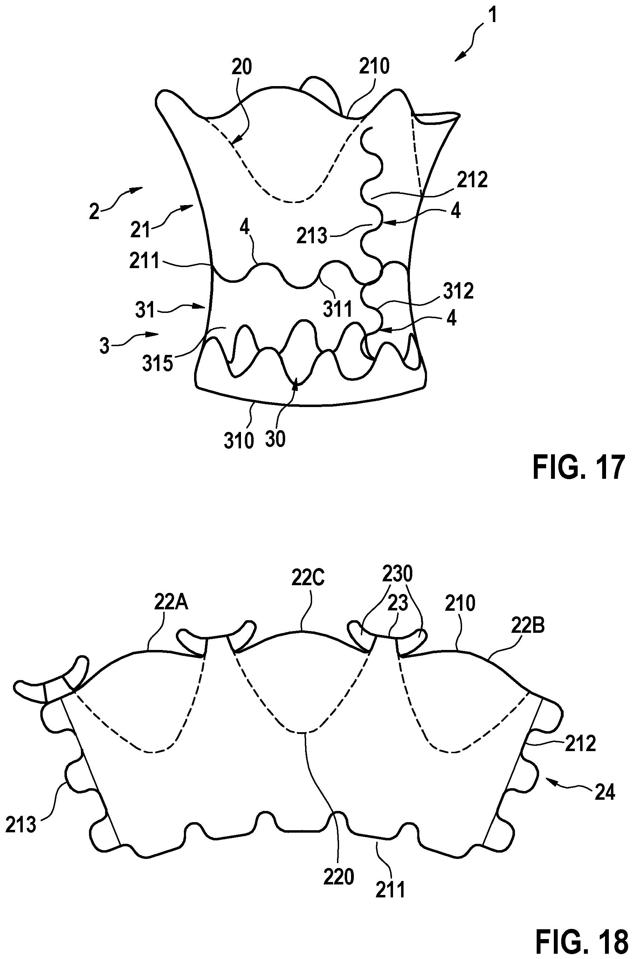

Referring now to Fig. 17, the valve member 2 and the anchoring member 3 are interconnected by means of a seam 4 for fixing the flexible leaflet arrangement 21 of the valve member 2 to the flexible skirt arrangement 31 of the anchoring member 3. In addition, one or multiple seams 4 are formed on the flexible leaflet arrangement 21, respectively, the flexible skirt arrangement 31 in order to form in each case a circumferentially closed structure by means of one or multiple pieces of material for forming the valve member 2, respectively, the anchoring member 3.

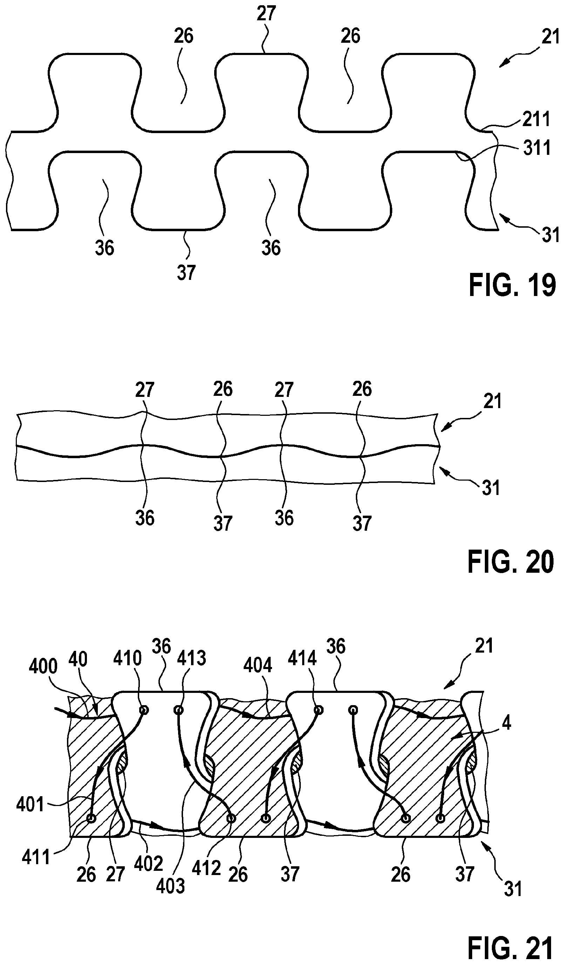

Referring now to Fig. 18, a piece of material 24 forming the flexible leaflet arrangement 21 may, at a joining edge 211 as well as at lateral edges 212, 213, have a meandering shape formed by, as visible in Fig. 19, joining flaps 26 alternating with recesses 27.

Just as well, a piece of material 34 forming the flexible skirt arrangement 31, as illustrated in Fig. 11, at a joining edge 311 as well as at lateral edges 212, 213 may have a meandering shape formed by joining flaps 36 alternating with recesses 37, as also visible in Fig. 19.

Referring now to Figs. 19 and 20, in one embodiment, the flexible leaflet arrangement 21 of the valve member 2 and the flexible skirt arrangement 31 of the anchoring member 3 - after forming the circumferentially closed structure of the flexible leaflet arrangement 21 and the flexible skirt arrangement 32 and after fixing the flexible leaflet arrangement 21 and the flexible skirt arrangement 31 to the valvular support structure 20, respectively, the anchoring support structure 30 - are connected to each other by arranging the valve member 2 and the anchoring member 3 on each other such that the joining flaps 26, 36 of the flexible leaflet arrangement 21 and the flexible skirt arrangement 31 interleave with each other. The joining flaps 26 of the flexible leaflet arrangement 21 hence engage with recesses 37 of the flexible skirt arrangement 31 and the joining flaps 36 of the flexible skirt arrangement 31 engage with the recesses 27 of the flexible leaflet arrangement 21, as shown in Fig. 20.

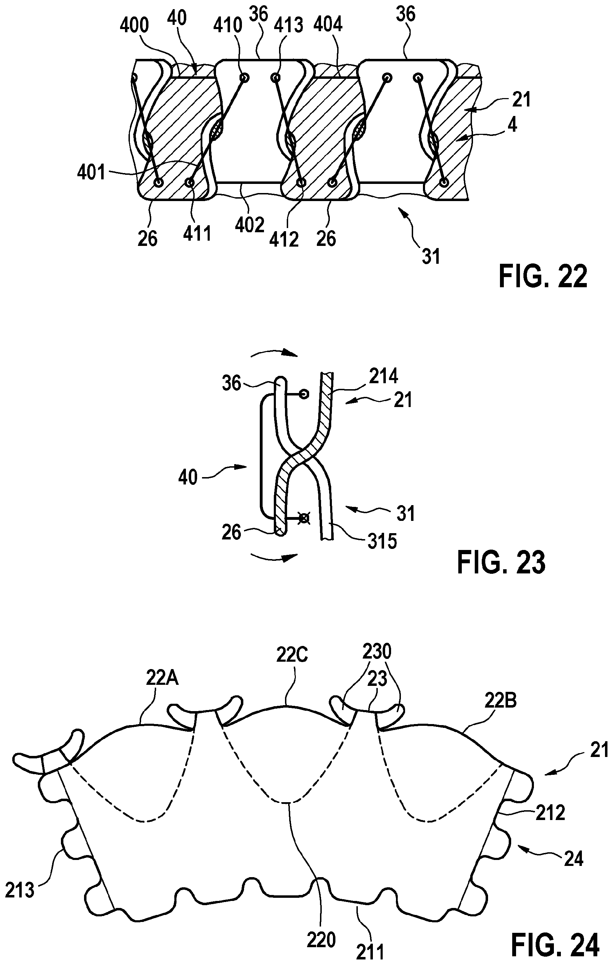

Herein, in one embodiment, the joining flaps 26, 36 are arranged on one another to interleave with one another such that the joining flaps 26, 36 are arranged at an outer side of the heart valve prosthesis 1, the joining flaps 26, 36 coming to rest at an outside of a body 214 of the flexible leaflet arrangement 21 and a body 315 of the flexible skirt arrangement 31, as this is visible from Fig. 20 in view of Figs. 21 and 23. In this way, the interleaving joining flaps 26, 36 may provide for a fixed, tight connection in between the valve member 2 and the anchoring member 3, without, however, interfering with a flow path inside of the heart valve prosthesis 1 and hence without altering a flow through the heart valve prosthesis 1.

Referring now to Figs. 21 to 23, a seam 4 for interconnecting the valve member 2 and the anchoring member 3 is formed by a thread 40 forming thread sections 400-404, the seam 4 having an opened trapezoidal shape. Herein, in contrast to Fig. 20 which shows the flexible leaflet arrangement 21 and the flexible skirt arrangement 31 from the inside of the heart valve prosthesis 1, Figs. 21 and 22 show the flexible leaflet arrangement 21 and the flexible skirt arrangement 31 at the location of the seam 4 from the outside of the heart valve prosthesis 1. The seam 4 is solely formed on the outside and, as visible from Fig. 20, does not extend to the inside and hence does not interfere with a blood flow on the inside of the heart valve prosthesis 1.
Whereas Fig. 21 shows the seam 4 with a thread 40 forming the seam 4 in a loose state, Fig. 22 shows the seam 4 with the thread 40 forming the seam 4 being tightened, as it is the case in an assembled state of the heart valve prosthesis 1.

In one embodiment, transverse thread portions 400, 402, 404 extend in between neighboring joining flaps 26, 36, each thread portion 400, 402, 404 being placed at an outside of the heart valve prosthesis 1, the thread portions 400, 402, 404 extending in parallel to one another as visible from Figs. 21 and 22.

Each transverse thread portion 400, 402, 404 connects alike joining flaps 26, 36. In particular, thread portion 402 extends in between two adjacent joining flaps 26 of the flexible leaflet arrangement 21. Thread portion 404 extends in between two adjacent joining flaps 36 of the flexible skirt arrangement 31.

Each transverse thread portion 400, 402, 404 is adjoined by oblique thread portions 401, 403, the oblique thread portions 401, 403 extending likewise at the outside of the flexible leaflet arrangement 21 and the flexible skirt arrangement 31, as visible from Figs. 21 and 22, such that the entire thread 40 forming the seam 4 comes to rest outside of the inner lumen of the heart valve prosthesis 1. Herein, thread portion 400 extends towards a thread opening 410 formed in a flap section 36, extends through the thread opening 410 and is adjoined by thread portion 401, which extends across the outside of the associated flap sections 36, 26 towards a thread opening 411 formed in the flap section 26 and through the thread opening 411 and is adjoined by thread portion 402, which extends across neighboring joining flaps 26 at the outside of the flexible leaflet arrangement 21 and the flexible skirt arrangement 31 towards a thread opening 412 formed in the neighboring flap section 26. At the thread opening 412 the thread 40 passes towards the outside of the flap section 26, as visible in Fig. 21, thread portion 403 extending at the outside of the heart valve prosthesis 1 towards thread opening 413 formed in the associated flap section 36. The thread 40 extends through the thread opening 413, such that thread portion 404 extends at the outside between neighboring flaps 36 towards thread opening 414, and so on.

Due to the formation of the seam 4 by means of the thread 40, oblique portions 401, 403 as well as transverse portions 400, 402, 404 are placed at the outside of the flexible leaflet arrangement 21 and the flexible skirt arrangement 31, hence allowing for an unaltered flow through the heart valve prosthesis 1, because the seam 4 does not interfere with a flow at the inside of the heart valve prosthesis 1.

In addition, due to the formation of the seam 4 and because the flap sections 26, 36 are placed at the outside and generally may abut with tissue surrounding the heart valve prosthesis 1, the joining flaps 26, 36 may tightly abut with the body 315 of the flexible skirt arrangement 31, respectively, the body 214 of the flexible leaflet arrangement 21, such that the joining flaps 26, 36 provide for a fixed interconnection while preventing an irregular bulging of material at the location of the seam 4.

Referring now to Figs. 24 to 41, for manufacturing the heart valve prosthesis 1, the valve member 2 and the anchoring member 3 may be formed first at separate units, and may then be mounted on each other to complete the heart valve prosthesis 1.

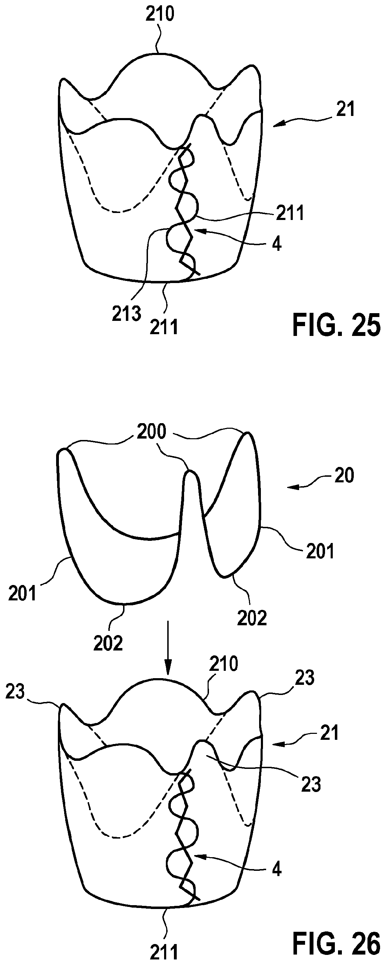

Referring first to Figs. 24 to 32, for manufacturing the valve member 2 the circumferentially closed structure of the flexible leaflet arrangement 21 is formed for example from a single piece of material 24 forming the leaflets 22A, 22B, 22C (Fig. 24) by forming a seam 4 at edges 212, 213 (Fig. 25).

Once the closed structure of the flexible leaflet arrangement 21 is formed, the valvular support structure 20 is placed inside the flexible leaflet arrangement 21 (Fig. 26), the valvular support structure 20 being received within the flexible leaflet arrangement 21 such that the valvular support structure 20 comes to rest with upper vertices 200 in the region of the fastening sections 23 of the flexible leaflet arrangement 21.

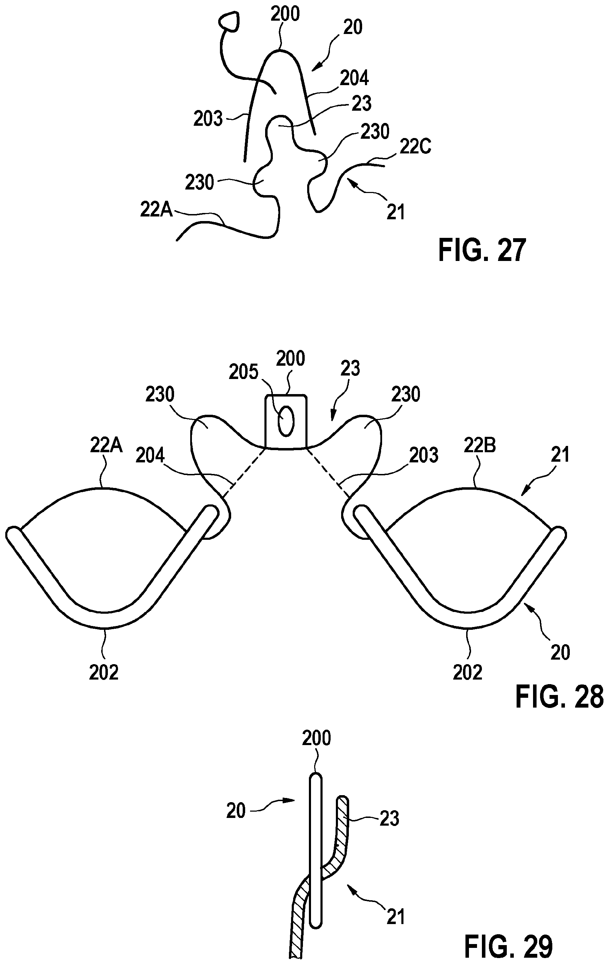

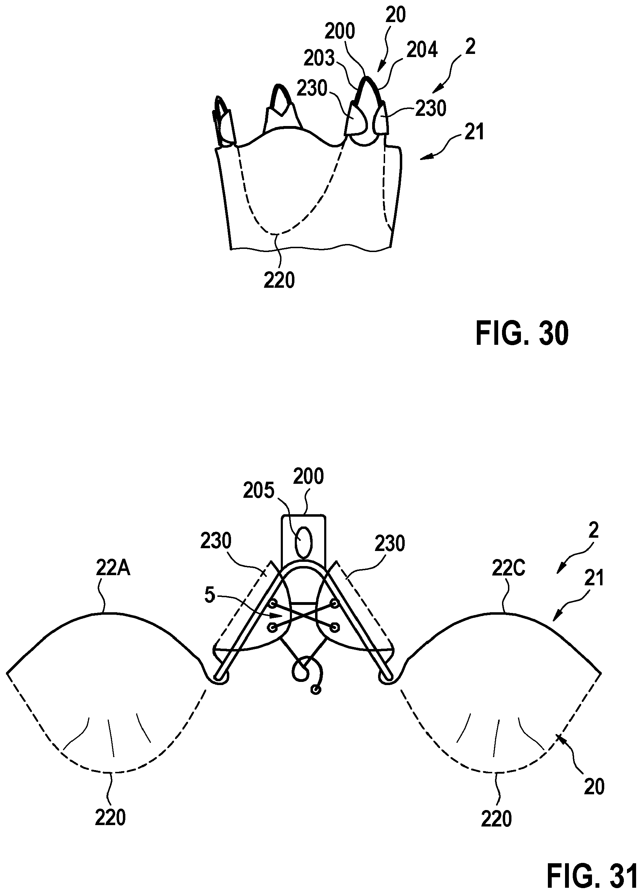

Subsequently, as illustrated in Figs. 27 to 32, the fastening sections 23 are fixed to the associated tip sections formed at the upper vertices 200 in that each fastening section 23, from the outside, is inserted through beam sections 203, 204 adjoining the associated upper vertex 200 (Fig. 27) in order to then fold the flap sections 230 of the fastening section 23 back towards the inside, as illustrated in Figs. 28 to 30. The flap sections 230 hence, as visible from Fig. 30, reach to the outside of the tip sections formed by the upper vertices 200 and the adjoining beam sections 203, 204 of the valvular support structure 20, allowing hence to sew together the flap sections 230 by means of for example a cross-shaped seam connection 5, as illustrated in Fig. 31.

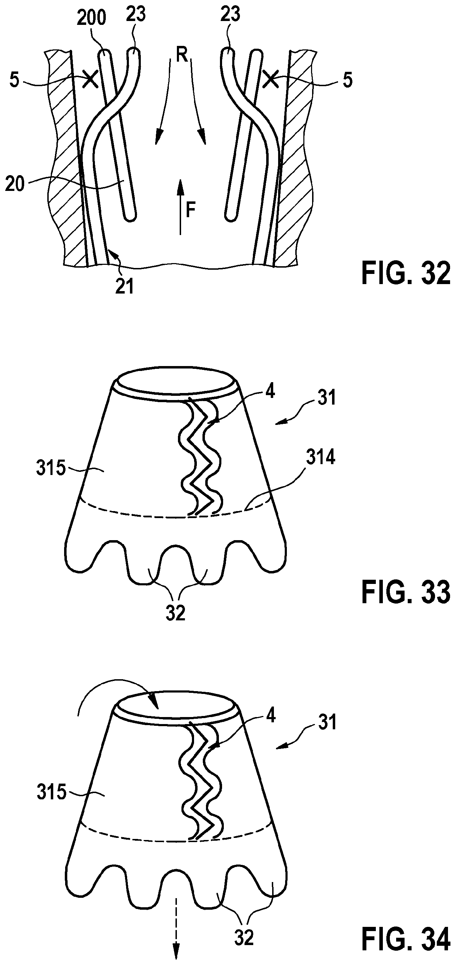

The seam connection 5 hence, as illustrated in Fig. 32, comes to rest outside of the valvular support structure 20, and hence is arranged outside of a flow path formed within the valve member 2, such that the seam connection 5 does not interfere with a flow through the valve member 2.

Referring now to Figs. 33 to 40, for manufacturing the anchoring member 3 the circumferentially closed structure of the skirt arrangement 31 is formed for example from a single piece of material by sewing lateral edges of the skirt arrangement 31 together to form a seam 4 (Fig. 33).

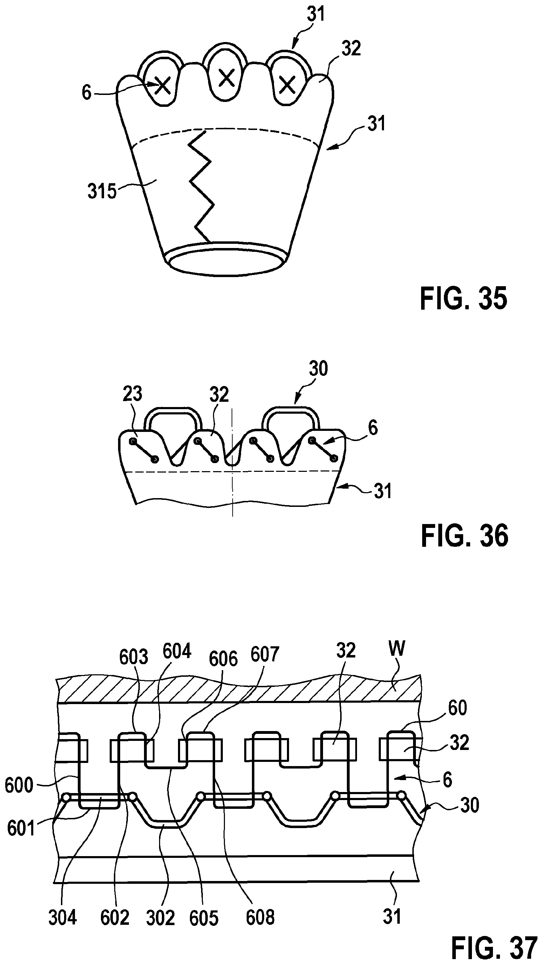

The flexible skirt arrangement 31 now is turned inside-out (Fig. 34) in order to fix the anchoring support structure 30 to the folding sections 32 of the flexible skirt arrangement 31 (Fig. 35).
The fixation of the anchoring support structure 30 to the folding sections 32 of the flexible skirt arrangement 31 takes place by means of a seam 6 which, as schematically shown in Fig. 37, interconnects the anchoring support structure 30 to the folding sections 32.

As visible from Fig. 37, the seam 6 is formed by a thread 60 forming thread sections 600-608.

In particular, a thread section 600 extends from a folding section 32 towards the anchoring support structure 30. An adjoining thread section 601 extends around the anchoring support structure 30, and a thread section 602 extends back towards a neighboring folding section 32. A thread section 603 extends across the folding section 32, a thread section 604 extends through the folding section 32, a thread section 605 extends across towards another, neighboring folding section 32, a thread section 606 extends through the folding section 32 towards the outside, a thread section 607 extends across the respective folding section 32, and a thread section 608 extends through the folding section 32 and towards another section of the anchoring support structure 30. In this way, the anchoring support structure 30 is fixedly connected to the folding sections 32 and in this way to the flexible skirt arrangement 31.

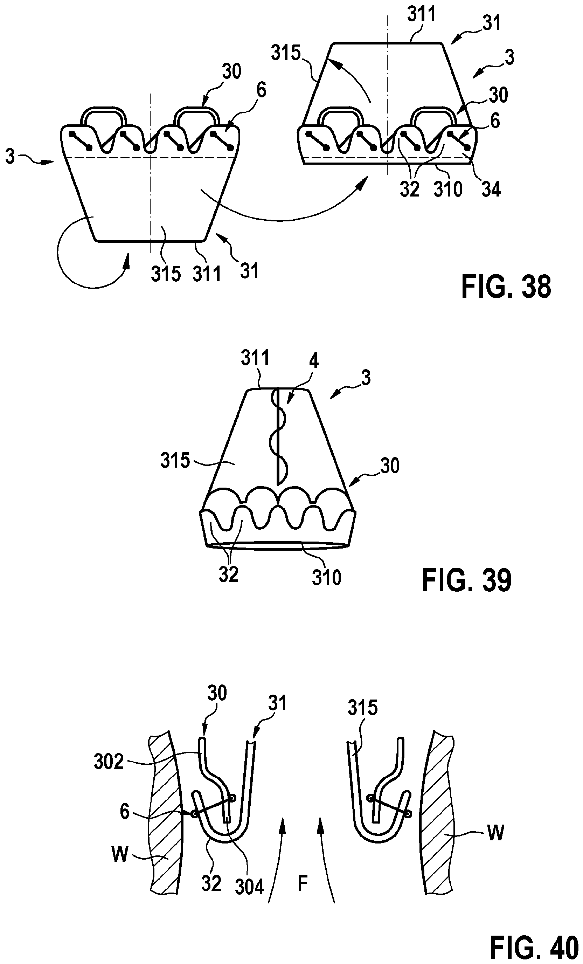

As visible from Fig. 38, now the flexible skirt arrangement 31 with the anchoring support structure 30 fixed thereto is folded back in that the body 315 is turned outside-in through the anchoring support structure 30 such that the anchoring member 3 is formed, as shown in Fig. 39.

In this way, the anchoring support structure 30, at the radially inner portions 304, by means of a seam connection 6 is connected to the flexible skirt arrangement 31. The anchoring support structure 30 herein is sewed at the radially inner portions 304 to the folding sections 32 folded with respect to the body 315 of the flexible skirt arrangement 31 to reach around the radially inner portions 304 of the anchoring support structure 30, as visible from Fig. 40.

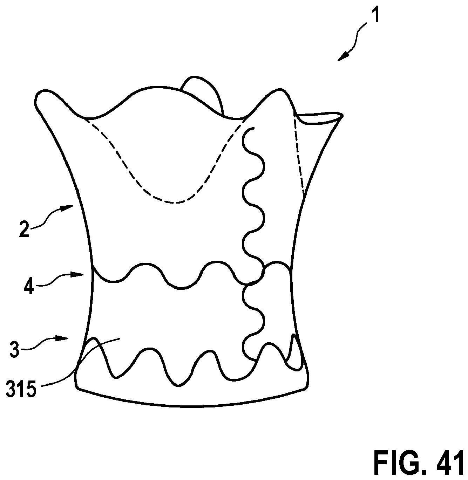

By now connecting the valve member 2 and the anchoring member 3 to one another by means of the seam 4, the heart valve prosthesis 1 is formed, as shown in Fig. 41, the heart valve prosthesis 1 being deployable by means of a delivery catheter in that the heart valve prosthesis 1 may be received within the delivery catheter in a radially compressed state and may be deployed by self-expansion at an implantation site to engage with native structure at the implantation site.

The invention is not limited to the embodiments described above, but may be implemented in an entirely different fashion.

A heart valve prosthesis of the kind described above may be used as a prosthetic valve replacement at an aortic valve of a patient, but in principle may also be used as a valve at a different location. For example, the heart valve prosthesis may be used as a mitral valve prosthesis or a tricuspid valve prosthesis.

Structure, design and shape of a valve member and an anchoring member may differ from the embodiments described above. In particular, different numbers of leaflets may be used on a valve member, and accordingly a shape of a valvular support structure may differ from embodiments described above. Likewise, a design of an anchoring support structure may differ from embodiments described above.

### List of reference numerals

- 1: Heart valve prosthesis
- 2: Valve member
- 20: Valvular support structure (support ring)
- 200: Tip section (Upper vertex)
- 201: Curved section
- 202: Lower vertex
- 203, 204: Beam section
- 205: Eyelet
- 21: Leaflet arrangement
- 210: Outflow edge
- 211: Joining edge
- 212,213: Edge
- 214: Body
- 22A, 22B, 22C: Leaflet
- 220: Bending line
- 23: Fastening section
- 230: Flap section
- 24: Material
- 25: Wire
- 250, 251: End
- 252, 253: Flattened section
- 254: Slot arrangement
- 255: Positive-locking arrangement
- 26: Joining flap
- 27: Recess
- 3: Anchoring member
- 30: Anchoring support structure
- 300: Upper vertex
- 301: Lower vertex
- 302: Outer portion
- 303: Linking portion
- 304: Inner portion
- 31: Skirt arrangement
- 310: Inflow edge
- 311: Joining edge
- 312, 313: Edge
- 314: Fold line
- 315: Body
- 32: Folding section
- 34: Material
- 36: Flap section
- 37: Recess
- 4: Seam
- 40: Thread
- 400-404: Thread portion
- 410-414: Thread opening
- 5: Seam
- 6: Seam
- 60: Thread
- 600-604: Thread portion
- F: Antegrade flow direction
- P: Pressure distribution
- R: Retrograde flow direction
- W: (Aortic) wall section

In view of all the foregoing disclosure, the present invention also provides for the following consecutively numbered embodiments:
1. A heart valve prosthesis (1), comprising:
   a valve member (2) having a flexible leaflet arrangement (21) and a valvular support structure (20) for supporting the flexible leaflet arrangement (21), the flexible leaflet arrangement (21) forming at least one flexible leaflet (22A, 22B, 22C) which is movable between an opened position for allowing a blood flow in an antegrade flow direction (F) and a closed position for blocking a blood flow in a retrograde flow direction (R); and
   an anchoring member (3) for anchoring the heart valve prosthesis (1) within a patient, the anchoring member (3) comprising a flexible skirt arrangement (31) and an anchoring support structure (30) for supporting the flexible skirt arrangement (31);
      **characterized in**
      that the valvular support structure (21) and the anchoring support structure (31) are structurally separate from one another, but are flexibly connected to each other via the flexible leaflet arrangement (21) and the flexible skirt arrangement (31).
2. The heart valve prosthesis (1) of embodiment 1, **characterized in** that the flexible leaflet arrangement (21) and/or the flexible skirt arrangement (31) are made from a biocompatible material, particularly from a biocompatible pericardial material of animal origin, preferably a bovine, porcine, horse or kangaroo pericardial material, a bacterial cellulose material or a polymeric material.
3. The heart valve prosthesis (1) of embodiment 1 or 2, **characterized in** that the flexible skirt arrangement (31) forms an inflow edge (310), wherein at least a portion of the flexible skirt arrangement (31) is conically tapered in the antegrade flow direction (F) beyond the inflow edge (310).
4. The heart valve prosthesis (1) of one of embodiments 1 to 3, **characterized in** that the flexible leaflet arrangement (21) forms an outflow edge (210), wherein at least a portion of the flexible leaflet arrangement (21) is conically tapered in the retrograde flow direction (R) beyond the outflow edge (310).
5. The heart valve prosthesis (1) of one of the preceding embodiments, **characterized in** that the valvular support structure (20) is formed by a ring element.
6. The heart valve prosthesis (1) of one of the preceding embodiments, **characterized in** that the valvular support structure (20) forms at least one curved section (201) for supporting the flexible leaflet arrangement (21), the at least one curved section (201) being associated with the at least one flexible leaflet (22A, 22B, 22C) for defining a bending line (220) about which the at least one flexible leaflet (22A, 22B, 22C) is flexibly bendable for a movement between the opened position and the closed position.
7. The heart valve prosthesis (1) of one of the preceding embodiments, **characterized in** that the valvular support structure (20) forms at least one tip section (200), wherein the flexible leaflet arrangement (21) is fixed to the at least one tip section (200).
8. The heart valve prosthesis (1) of one of the preceding embodiments, **characterized in** that the anchoring support structure (30) is formed by a ring element.
9. The heart valve prosthesis (1) of one of the preceding embodiments, **characterized in** that the anchoring support structure (30) has a meandering shape.
10. The heart valve prosthesis (1) of one of the preceding embodiments, **characterized in** that the anchoring support structure (30) comprises a multiplicity of radially outer portions (302) and a multiplicity of radially inner portions (304), wherein, when viewed along a circumferential direction about the antegrade flow direction (F), the radially outer portions (302) alternate with the radially inner portions (304).
11. The heart valve prosthesis (1) of one of the preceding embodiments, **characterized in** that the flexible skirt arrangement (31) comprises a body (315) and at least one folding section (32) formed on the body (315) and folded, at a fold line (314), with respect to the body (315) to reach around the anchoring support structure (30).
12. The heart valve prosthesis (1) of one of the preceding embodiments, **characterized in** that the flexible leaflet arrangement (21) comprises a first joining edge (211) and the flexible skirt arrangement (31) comprises a second joining edge (311), wherein the first joining edge (211) and the second joining edge (311) are connected to each other (311) by a seam (4).
13. The heart valve prosthesis (1) of embodiment 12, **characterized in** that the first joining edge (211) and/or the second joining edge (311) comprise a meandering shape.
14. The heart valve prosthesis (1) of embodiment 12 or 13, **characterized in** that the flexible leaflet arrangement (21), at the first joining edge (211), forms at least one first joining flap (26) and/or the flexible skirt arrangement (31), at the second joining edge (311), forms at least one second joining flap (36).
15. The heart valve prosthesis (1) of one of the preceding embodiments, **characterized in** that the connection of the valvular support structure (21) and the anchoring support structure (31) via the flexible leaflet arrangement (21) and the flexible skirt arrangement (31) is releasable in an implanted state of the heart valve prosthesis (1).

## Claims

1. A heart valve prosthesis (1), comprising:
a valve member (2) having a flexible leaflet arrangement (21) and a valvular support structure (20) for supporting the flexible leaflet arrangement (21), the flexible leaflet arrangement (21) forming at least one flexible leaflet (22A, 22B, 22C) which is movable between an opened position for allowing a blood flow in an antegrade flow direction (F) and a closed position for blocking a blood flow in a retrograde flow direction (R); and
an anchoring member (3) for anchoring the heart valve prosthesis (1) within a patient, the anchoring member (3) comprising a flexible skirt arrangement (31) and
an anchoring support structure (30) for supporting the flexible skirt arrangement (31);
**characterized in**
**that** the valvular support structure (21) and the anchoring support structure (31) are structurally separate from one another, but are flexibly connected to each other via the flexible leaflet arrangement (21) and the flexible skirt arrangement (31).

2. The heart valve prosthesis (1) of claim 1, **characterized in that** the flexible leaflet arrangement (21) and/or the flexible skirt arrangement (31) are made from a biocompatible material, particularly from a biocompatible pericardial material of animal origin, preferably a bovine, porcine, horse or kangaroo pericardial material, a bacterial cellulose material or a polymeric material.

3. The heart valve prosthesis (1) of claim 1 or 2, **characterized in that** the flexible skirt arrangement (31) forms an inflow edge (310), wherein at least a portion of the flexible skirt arrangement (31) is conically tapered in the antegrade flow direction (F) beyond the inflow edge (310).

4. The heart valve prosthesis (1) of one of claims 1 to 3, **characterized in that** the flexible leaflet arrangement (21) forms an outflow edge (210), wherein at least a portion of the flexible leaflet arrangement (21) is conically tapered in the retrograde flow direction (R) beyond the outflow edge (310).

5. The heart valve prosthesis (1) of one of the preceding claims, **characterized in that** the valvular support structure (20) is formed by a ring element.

6. The heart valve prosthesis (1) of one of the preceding claims, **characterized in that** the valvular support structure (20) forms at least one curved section (201) for supporting the flexible leaflet arrangement (21), the at least one curved section (201) being associated with the at least one flexible leaflet (22A, 22B, 22C) for defining a bending line (220) about which the at least one flexible leaflet (22A, 22B, 22C) is flexibly bendable for a movement between the opened position and the closed position.

7. The heart valve prosthesis (1) of one of the preceding claims, **characterized in that** the valvular support structure (20) forms at least one tip section (200), wherein the flexible leaflet arrangement (21) is fixed to the at least one tip section (200).

8. The heart valve prosthesis (1) of one of the preceding claims, **characterized in that** the anchoring support structure (30) is formed by a ring element.

9. The heart valve prosthesis (1) of one of the preceding claims, **characterized in that** the anchoring support structure (30) has a meandering shape.

10. The heart valve prosthesis (1) of one of the preceding claims, **characterized in that** the anchoring support structure (30) comprises a multiplicity of radially outer portions (302) and a multiplicity of radially inner portions (304), wherein, when viewed along a circumferential direction about the antegrade flow direction (F), the radially outer portions (302) alternate with the radially inner portions (304).

11. The heart valve prosthesis (1) of one of the preceding claims, **characterized in that** the flexible skirt arrangement (31) comprises a body (315) and at least one folding section (32) formed on the body (315) and folded, at a fold line (314), with respect to the body (315) to reach around the anchoring support structure (30).

12. The heart valve prosthesis (1) of one of the preceding claims, **characterized in that** the flexible leaflet arrangement (21) comprises a first joining edge (211) and the flexible skirt arrangement (31) comprises a second joining edge (311), wherein the first joining edge (211) and the second joining edge (311) are connected to each other (311) by a seam (4).

13. The heart valve prosthesis (1) of claim 12, **characterized in that** the first joining edge (211) and/or the second joining edge (311) comprise a meandering shape.

14. The heart valve prosthesis (1) of claim 12 or 13, **characterized in that** the flexible leaflet arrangement (21), at the first joining edge (211), forms at least one first joining flap (26) and/or the flexible skirt arrangement (31), at the second joining edge (311), forms at least one second joining flap (36).

15. The heart valve prosthesis (1) of one of the preceding claims, **characterized in that** the connection of the valvular support structure (21) and the anchoring support structure (31) via the flexible leaflet arrangement (21) and the flexible skirt arrangement (31) is releasable in an implanted state of the heart valve prosthesis (1).
